# EUROPEAN PATENT APPLICATION

(11) **EP 4 851 184 A1**
(43) Date of publication of application: **09.12.2026**
(21) Application number: 26166112.8
(22) Date of filing: 19.03.2026
(51) Int. Cl.: C07K 14/605, A61K 38/00, A61K 38/26, A61K 47/54

(54) **GLP-1R AND GCGR DUAL-TARGET AGONIST PEPTIDE OR DERIVATIVE OR PHARMACEUTICALLY ACCEPTABLE SALT THEREOF, AND USES THEREOF**

(30) Priority: 05.06.2025 CN 202510743650
(71) Applicant: Tencent Technology (Shenzhen) Company Limited, Shenzhen, Guangdong 518057 (CN)
(72) Inventor: He, Bing, Shenzhen, 518057 (CN); Lyu, Tianxu, Shenzhen, 518057 (CN); Yao, Jianhua, Shenzhen, 518057 (CN); Zhao, Yu, Shenzhen, 518057 (CN); Qin, Chenchen, Shenzhen, 518057 (CN)
(74) Representative: Nederlandsch Octrooibureau

(57) **Abstract**

This application provides a peptide or a derivative or pharmaceutically acceptable salt thereof. An amino acid sequence of the peptide is set forth in SEQ ID NO: 1. The peptide or the derivative or pharmaceutically acceptable salt thereof in this application can be used for, through binding to GLP-1R and GCGR, effectively activating GLP-1R and GCGR. Accordingly, the peptide or the derivative or pharmaceutically acceptable salt thereof can be used for detecting GLP-IR and/or GCGR, as well as for treating or preventing GLP-1R and/or GCGR-related diseases (including, but not limited to, metabolic disorder-related diseases such as obesity, diabetes, fatty liver disease, and non-alcoholic fatty liver disease).

## Description

### FIELD OF THE TECHNOLOGY

This application belongs to the field of biopharmaceutical technology, and specifically relates to a GLP-1R and GCGR dual-target agonist peptide or a derivative or pharmaceutically acceptable salt thereof, and uses thereof.

### BACKGROUND OF THE DISCLOSURE

Glucagon-like peptide-1 (GLP-1) is a peptide composed of 31 amino acids, expressed by the proglucagon gene in intestinal mucosal L cells. GLP-1 primarily binds to the GLP-1 receptor (GLP-1R) to stimulate insulin secretion, inhibit glucagon secretion, protect pancreatic β-cells, and regulate blood glucose homeostasis. In addition, GLP-1 can also suppress appetite and gastric emptying through central nervous system signaling pathways, increase satiety, and thereby reduce body weight.

Glucagon (GCG) is a peptide containing 29 amino acids secreted by pancreatic α-cells. It mainly acts on the glucagon receptor (GCGR), which is predominantly distributed in the liver and kidneys. GCG can stimulate hepatic glycogen breakdown, elevate blood glucose levels, activate lipase, promote lipolysis, and enhance fatty acid oxidation, thereby increasing ketone body production. Research results indicate that GCG has certain effects in reducing food intake, increasing energy expenditure in adipose tissue, and decreasing body fat content.

In the related art, GLP-1 receptor (GLP-1R) agonists and glucagon receptor (GCGR) agonists have been extensively studied and applied. GLP-1 receptor agonists lower blood glucose by mimicking the action of endogenous GLP-1, while GCGR agonists influence blood glucose levels by regulating glucagon secretion.

However, these single-target agonists exhibit certain limitations in treating diabetes, such as short half-lives and susceptibility to degradation. Currently, in addition to the aforementioned single agonists targeting GLP-1R or GCGR, experiments suggest that several GLP-1R/GCGR dual agonists may offer more significant weight loss effects and demonstrate potential advantages in treating fatty liver diseases and related complications, exerting positive effects through synergistic regulation of glucose and lipid metabolism. Therefore, developing a novel GLP-1R and GCGR dual-target agonist peptide holds important utility in treating diabetes, obesity, and other related diseases.

### SUMMARY

This application aims to address at least one of the technical problems existing in the related art. For this purpose, this application provides a GLP-1R and GCGR dual-target agonist peptide.

In a first aspect of this application, this application provides a peptide or a derivative or pharmaceutically acceptable salt thereof. According to an embodiment of this application, an amino acid sequence of the peptide is set forth in SEQ ID NO: 1. The peptide or the derivative or pharmaceutically acceptable salt thereof in this application can be used for, through binding to GLP-1R and GCGR, effectively activating GLP-1R and GCGR. Accordingly, the peptide or the derivative or pharmaceutically acceptable salt thereof can be used for detecting GLP-1R and/or GCGR, as well as for treating or preventing GLP-1R and/or GCGR-related diseases (including, but not limited to, metabolic disorder-related diseases, such as dyslipidemia-related disease, for example, metabolic syndrome, especially obesity, diabetes, fatty liver disease, and non-alcoholic fatty liver disease).

According to an embodiment of this application, the peptide or the derivative or pharmaceutically acceptable salt thereof may further include at least one of the following technical features: According to an embodiment of this application, the derivative of the peptide includes a modifying group, and the peptide is linked to the modifying group.

According to an embodiment of this application, the modifying group is linked to -NH₂ of an amino acid side chain in the peptide.

According to an embodiment of this application, the modifying group has at least one of the following structures:

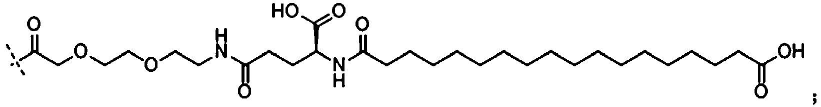

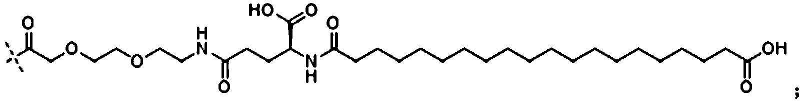

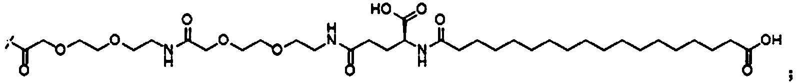

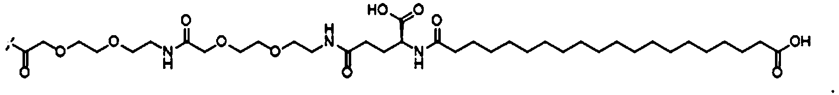

According to a second aspect of this application, this application provides use of the peptide or the derivative or pharmaceutically acceptable salt thereof according to the first aspect in preparation of a GLP-1R and GCGR dual-target agonist. Accordingly, the peptide or the derivative or pharmaceutically acceptable salt thereof is effective in activating GLP-1R and GCGR. Accordingly, the peptide or the derivative or pharmaceutically acceptable salt thereof can be prepared as a GLP-1R and GCGR dual-target agonist for treating or preventing a GLP-1R and/or GCGR-related disease, including, but not limited to, metabolic disorder-related diseases, such as dyslipidemia-related disease, for example, metabolic syndrome, especially obesity, diabetes, fatty liver disease, and non-alcoholic fatty liver disease.

In a third aspect of this application, this application provides a fusion protein. According to an embodiment of this application, the fusion protein includes the peptide or the derivative or pharmaceutically acceptable salt thereof according to the first aspect. Accordingly, the peptide or the derivative or pharmaceutically acceptable salt thereof can be used for, through binding to GLP-1R and GCGR, effectively activating GLP-1R and GCGR. Accordingly, the fusion protein containing the peptide or the derivative or pharmaceutically acceptable salt thereof can be used for, through binding to GLP-1R and GCGR, detecting GLP-1R and/or GCGR or activating GLP-1R and GCGR, as well as treating or preventing GLP-1R and/or GCGR-related diseases, including, but not limited to, metabolic disorder-related diseases, such as dyslipidemia-related disease, for example, metabolic syndrome, especially obesity, diabetes, fatty liver disease, and non-alcoholic fatty liver disease.

In a fourth aspect of this application, this application provides a reagent or kit. According to an embodiment of this application, the reagent or kit includes the peptide or the derivative or pharmaceutically acceptable salt thereof according to the first aspect, or the fusion protein according to the third aspect. Accordingly, the peptide or the derivative or pharmaceutically acceptable salt thereof can be used for, through binding to GLP-1R and GCGR, effectively activating GLP-1R and GCGR. Accordingly, the reagent or kit containing the peptide or the derivative or pharmaceutically acceptable salt thereof can be used for, through binding to GLP-1R and GCGR, detecting GLP-1R and/or GCGR.

In a fifth aspect of this application, this application provides a pharmaceutical composition. According to an embodiment of this application, the pharmaceutical composition includes the peptide or the derivative or pharmaceutically acceptable salt thereof according to the first aspect, or the fusion protein according to the third aspect. Accordingly, the peptide or the derivative or pharmaceutically acceptable salt thereof can be used for, through binding to GLP-1R and GCGR, effectively activating GLP-1R and GCGR. Accordingly, the pharmaceutical composition including the peptide can be used for treating or preventing GLP-1R and/or GCGR-related diseases, including, but not limited to, metabolic disorder-related diseases, such as dyslipidemia-related disease, for example, metabolic syndrome, especially obesity, diabetes, fatty liver disease, and non-alcoholic fatty liver disease.

According to an embodiment of this application, the pharmaceutical composition may further include a pharmaceutically acceptable excipient.

In a sixth aspect of this application, this application provides use of the peptide or the derivative or pharmaceutically acceptable salt thereof according to the first aspect, the fusion protein according to the third aspect, or the pharmaceutical composition according to the fifth aspect in preparation of a medicament for treating or preventing a GLP-1R and/or GCGR-related disease, such as metabolic disorder-related disease.

According to an embodiment of this application, the GLP-1R and/or GCGR-related disease includes a metabolic disorder-related disease.

According to an embodiment of this application, the metabolic disorder-related disease includes a dyslipidemia-related disease.

According to an embodiment of this application, the dyslipidemia-related disease includes a metabolic syndrome.

According to an embodiment of this application, the metabolic syndrome includes at least one of obesity, diabetes, fatty liver disease, and non-alcoholic fatty liver disease.

In a seventh aspect of this application, this application provides a method for detecting GLP-1R and/or GCGR. According to an embodiment of this application, the method includes: contacting a sample to be detected with the peptide or the derivative or pharmaceutically acceptable salt thereof according to the first aspect, the fusion protein according to the third aspect, or the reagent or kit according to the fourth aspect; and determining whether the sample to be detected contains GLP-1R and/or GCGR based on a signal generated from a contact product. Accordingly, the peptide or the derivative or pharmaceutically acceptable salt thereof can be used for, through binding to GLP-1R and GCGR, effectively activating GLP-1R and GCGR. Accordingly, the method can be used for, through binding to GLP-1R and GCGR, detecting GLP-1R and/or GCGR.

Other aspects and advantages of this application will be given in the following description, some of which will become apparent from the following description or may be learned from practices of this application.

### BRIEF DESCRIPTION OF THE DRAWINGS

The foregoing and/or additional aspects and advantages of this application will become apparent and comprehensible in the description of the embodiments made with reference to the following accompanying drawings.
FIG. 1 shows a schematic diagram of a peptide design and screening process according to this application.
FIG. 2 shows detection results of cAMP release upon activation of a GLP-1R target by GA-Dual.R5.S3 in Embodiment 2 of this application.
FIG. 3 shows detection results of luciferase release upon activation of a GCGR target by GA-Dual.R5.S3 in Embodiment 2 of this application.

### DESCRIPTION OF EMBODIMENTS

The terms "first" and "second" are used for descriptive purposes only and are not to be construed as indicating or implying relative importance or implicitly specifying the quantity of the indicated technical features. Thus, a feature defined with "first" or "second" may explicitly or implicitly include one or more such features. Further, in the description of this application, unless otherwise specified, the term "multiple" means two or more.

Herein, the terms "comprise" or "include" are open-ended expressions, i.e., they encompass what is specified in this application but do not exclude other aspects.

Herein, the terms "in some embodiments", "in an embodiment", or "an embodiment" generally mean that the subsequently described event or circumstance may, but need not, occur, and the description includes both the situation in which the event or circumstance occurs and the situation in which it does not occur.

Herein, the term "target protein" refers to a protein that plays a key role in a living organism and is usually the object of action in medicament development. Through binding to the target protein, a medicament can regulate its biological activity, thereby achieving the purpose of treating diseases. Herein, the term "peptide" refers to a biological macromolecule composed of multiple amino acids, usually 50 or fewer amino acids linked together by peptide bonds. Peptides serve various functions in living organisms, including participating in diverse physiological processes as enzymes, hormones, antibodies, and the like. Further, due to their favorable biocompatibility, selectivity, and high biological activity, peptides have been widely studied for use in medicament discovery and the treatment of various diseases, such as metabolic disorder-related diseases.

Herein, the term "dual-target peptide" refers to a peptide molecule capable of simultaneously binding to two different targets, thereby providing unique advantages in medicament development by enabling the simultaneous regulation of multiple biological pathways to enhance therapeutic efficacy.

Herein, the term "cyclic adenosine monophosphate (cAMP)" refers to a small molecule ubiquitous in living organisms, which is converted from adenosine triphosphate (ATP) through the catalytic action of adenylate cyclase. As a key intracellular signaling mediator, cAMP plays a crucial role in regulating a variety of cellular functions and physiological processes.

Herein, the term "peptide bio-activity" refers to the biological functions and activities exerted by a peptide within a living organism. A peptide can, through binding to a specific target protein, modulate biological processes such as signal transduction, immune responses, cell proliferation, and the like. The bio-activity of a peptide depends on its amino acid sequence, structure, and interaction with the target. Owing to their favorable biocompatibility and selectivity, peptides possess significant potential in medicament development, disease treatment, and biotechnological applications.

Herein, the term "EC₅₀" refers to the concentration of a peptide or a derivative or pharmaceutically acceptable salt thereof required to achieve 50% of the maximum response under specific experimental conditions. The response may be a physiological reaction of a living organism, cell growth, enzyme activity, and the like. EC₅₀ values are widely used in medicament development, toxicological studies, and biological experiments. By comparing the EC₅₀ values of different medicaments, their relative potency can be evaluated. A lower EC₅₀ value indicates higher potency of a medicament, as a significant biological response can be elicited at a lower concentration. EC₅₀ is typically determined from a dose-response curve. This curve depicts the effect of different concentrations of a medicament on the biological response, usually exhibiting a sigmoidal shape. By analyzing this curve, the concentration at which 50% of the maximum response is produced can be identified.

Herein, amino acids are designated using the conventional one-letter and three-letter codes for natural amino acids, as well as the generally accepted three-letter codes for other α-amino acids. Unless explicitly indicated otherwise, in this application, upper-case letters denote L-configured amino acids, and lower-case letters denote D-configured amino acids.

Herein, the structure of the term "αMeK" is

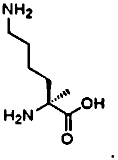

Herein, the structure of the term "HoK" is

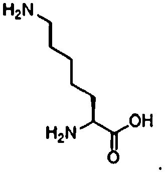

Herein, the structure of the term "N-Me-K" is

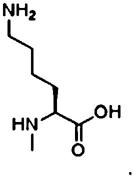

Herein, the structure of the term "Orn" is

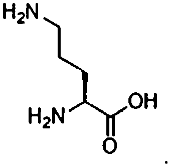

Herein, the structure of the term "Dab" is

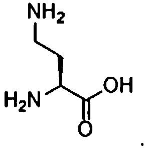

Herein, the structure of the term "Dap" is

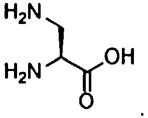

Herein, the term "pharmaceutically acceptable" refers to that the substance or composition need to be be chemically and/or toxicologically compatible with other ingredients including the peptide or the derivative thereof and/or with the mammal to be treated therewith. Preferably, "pharmaceutically acceptable" as used in this application refers to approval by a federal regulatory authority or national government, or listing in the United States Pharmacopeia or other generally recognized pharmacopoeia for use in animals, particularly in humans.

Herein, the term "pharmaceutically acceptable salt" refers to organic and inorganic salts of the peptide or the derivative thereof in this application. Pharmaceutically acceptable salts are well known in the art. Salts formed with pharmaceutically acceptable, non-toxic acids include, but are not limited to, inorganic acid salts formed by reaction with an amino group (e.g., hydrochloride, hydrobromide, phosphate, sulfate, perchlorate) and organic acid salts (e.g., acetate, oxalate, maleate, tartrate, citrate, succinate, malonate), or such salts obtained by other methods described in the literature, such as ion exchange.

Herein, the term "pharmaceutical composition" may refer to a composition for treating a disease or for use in in-vitro cell culture experiments. When used for treating a disease, the term "pharmaceutical composition" generally refers to a unit dosage form, and may be prepared by any of the methods well known in the pharmaceutical field. All methods include the operation of combining the active ingredient with excipients that constitute one or more accessory ingredients. Typically, the composition is prepared by uniformly and thoroughly combining the active peptide or the derivative thereof with a liquid excipient, a finely divided solid excipient, or both.

Herein, the term "pharmaceutically acceptable excipient" may include any solvent, solid excipient, diluent, or other liquid excipient suitable for an intended dosage form. The use of such excipients is also contemplated herein, except where any conventional excipient is incompatible with the peptide or the derivative thereof in this application, the pharmaceutical composition, or the medicament containing them, for instance, by producing any undesirable biological effect or interacting in a deleterious manner with any other component of the pharmaceutically acceptable composition.

The pharmaceutical composition in the present disclosure includes formulations suitable for parenteral administration. The formulations may conveniently be in unit dosage forms and can be prepared by any method known in the pharmaceutical field. The amount of active ingredient that can be combined with excipient materials to produce a single-dosage form is generally the amount of the peptide or the derivative thereof that produces a therapeutic effect.

Herein, the term "agonist" refers to a substance (ligand) that activates the receptor type.

Herein, the term "treatment" refers to achieving a desired pharmacological and/or physiological effect. The effect may be preventive in terms of completely or partially preventing a disease or its symptoms, and/or therapeutic in terms of partially or completely curing the disease and/or adverse effects caused by the disease. As used herein, "treatment" encompasses diseases in mammals, particularly humans, including: (a) preventing the onset of a disease or disorder in an individual prone to the disease but not yet diagnosed; (b) suppressing the disease, e.g., arresting its development; or (c) alleviating the disease, e.g., reducing symptoms associated with the disease. As used herein, "treatment" encompasses any administration of the peptide or the derivative or pharmaceutically acceptable salt thereof, or the medicament containing the same, to an individual to treat, cure, alleviate, ameliorate, reduce, or inhibit a disease of the individual, including but not limited to administering the medicament containing the peptide or the derivative or pharmaceutically acceptable salt thereof as described herein, to an individual in need thereof.

Herein, the term "non-alcoholic fatty liver disease (NAFLD)" generally refers to a clinicopathological syndrome primarily characterized by excessive fat deposition in hepatocytes, excluding alcohol and other clear liver-damaging factors, and is an acquired metabolic stress-induced liver injury closely related to insulin resistance and genetic predisposition, including but not limited to simple fatty liver (SFL), non-alcoholic steatohepatitis (NASH), and related cirrhosis.

This application provides a GLP-1R and GCGR dual-target agonist peptide or a derivative or pharmaceutically acceptable salt thereof, and uses thereof, which will be described in detail below.

Peptide or Derivative or Pharmaceutically Acceptable Salt Thereof

In one aspect of this application, this application provides a peptide or a derivative or pharmaceutically acceptable salt thereof. According to an embodiment of this application, an amino acid sequence of the peptide is set forth in SEQ ID NO: 1.

The inventors of this application have obtained a de novo GLP-1R and GCGR dual-target peptide through precise computation, deep-learning models, and iterative evolutionary model optimization. Compared with traditional GLP-1 and GCGR agonists, it possesses a unique amino acid arrangement. Further, the above-described peptide or the derivative or pharmaceutically acceptable salt thereof can, through binding to GLP-1R and GCGR, effectively activate GLP-1R and GCGR, and exhibit high affinity and bio-activity toward GLP-1R and GCGR. Accordingly, the peptide or the derivative or pharmaceutically acceptable salt thereof can be used for detecting GLP-1R and/or GCGR, as well as for treating or preventing GLP-1R and/or GCGR-related diseases (including, but not limited to, metabolic disorder-related diseases, such as dyslipidemia-related disease, for example, metabolic syndrome, especially obesity, diabetes, fatty liver disease, and non-alcoholic fatty liver disease).

In particular, with the rising global prevalence of metabolic disorder-related diseases such as diabetes and the limitations of existing treatment options, the peptide or the derivative or pharmaceutically acceptable salt thereof in this application can not only provide new treatment options for patients with diabetes and the like, but also holds the potential to reshape the market landscape for the treatment of diabetes and similar conditions.

Herein, the phrase "an amino acid sequence of the peptide is set forth in SEQ ID NO: A" includes the amino acid sequence of SEQ ID NO: A, a conservatively modified amino acid sequence of SEQ ID NO: A, or an amino acid sequence having sequence similarity of 90% or more (e.g., 90% or more, 91% or more, 92% or more, 93% or more, 94% or more, 95% or more, 96% or more, 97% or more, 98% or more, 99% or more) to the amino acid sequence set forth in SEQ ID NO: A, all of which fall within the scope of protection of this application. Unless otherwise specified, the conservatively modified amino acids or similarity-differentiating amino acids in "a conservatively modified amino acid sequence of SEQ ID NO: A, or an amino acid sequence having sequence similarity of 90% or more (e.g., 90% or more, 91% or more, 92% or more, 93% or more, 94% or more, 95% or more, 96% or more, 97% or more, 98% or more, 99% or more) to the amino acid sequence set forth in SEQ ID NO: A" may be located at any position in the peptide in this application. They may be located in at least one of positions 2, 4 to 12, 14, 22, and 25 to 26; or alternatively, in at least one of positions 1, 3, 13, 15 to 21, 23 to 24, and 27 to 30. Such amino acid modifications or similarity differences do not significantly affect or alter the structural stability of the peptide including the amino acids and/or its binding activity to the receptor, and all fall within the scope of protection of this application.

Exemplarily, "an amino acid sequence of the peptide is set forth in SEQ ID NO: 1" means that the peptide is an amino acid sequence set forth in SEQ ID NO: 1, a conservatively modified amino acid sequence of SEQ ID NO: 1, or an amino acid sequence having sequence similarity of 90% or more (e.g., 90% or more, 91% or more, 92% or more, 93% or more, 94% or more, 95% or more, 96% or more, 97% or more, 98% or more, 99% or more) to the amino acid sequence set forth in SEQ ID NO: 1, all of which fall within the scope of protection of this application.

Herein, the "conservatively modified amino acid sequence" refers to an amino acid modification that does not significantly affect or alter the properties of the sequence containing it; such modifications include amino acid substitutions, additions, and deletions. The modifications may be introduced into the peptide in this application by standard techniques such as site-directed mutagenesis and PCR-mediated mutagenesis. A conservative amino acid substitution is one in which an amino acid residue is substituted with another amino acid residue having a similar side chain. Families of amino acid residues having similar side chains have been defined in the art. These families include amino acids with basic side chains (e.g., lysine, arginine, histidine), amino acids with acidic side chains (e.g., aspartic acid, glutamic acid), amino acids with uncharged polar side chains (e.g., glycine, asparagine, glutamine, serine, threonine, tyrosine, cysteine, tryptophan), amino acids with nonpolar side chains (e.g., alanine, valine, leucine, isoleucine, proline, phenylalanine, methionine), amino acids with β-branched side chains (e.g., threonine, valine, isoleucine), and amino acids with aromatic side chains (e.g., tyrosine, phenylalanine, tryptophan, histidine). Exemplarily, the number of conservative modifications does not exceed 10% of the total number, preferably does not exceed 5% of the total number. Herein, the "conservatively modified amino acid sequence" also includes amino acid modifications arising from natural mutations, where "natural mutation" refers to mutations caused by changes such as alleles during the natural mutation process of the peptide.

Herein, the term "sequence similarity" is defined by a percentage similarity method, i.e., it is calculated by comparing the number of identical or similar amino acids in two protein or peptide sequences to the total number of amino acids, $% similarity = \left(\frac{Number of identical or similar amino acids}{Total number of amino acids}\right) \times 100 .$

According to an embodiment of this application, the peptide has an amino acid sequence set forth in SEQ ID NO: 1, or an amino acid sequence having at least 80% sequence similarity thereto; or, compared with the amino acid sequence set forth in SEQ ID NO: 1, the peptide is modified by substitution, deletion, or addition of 1 to 3 amino acids and possesses GLP-1R and GCGR binding activity, such as substitution, deletion, or addition of 1, 2, or 3 amino acids.

Herein, "by substitution, deletion, or addition of one or several amino acids" means that after such substitution, deletion, or addition of such amino acids, the binding properties of the original amino acid sequence are not significantly affected or altered. An amino acid substitution refers to the substitution of an amino acid residue in the original peptide chain with another amino acid residue having a similar side chain. Families of amino acid residues having similar side chains have been defined in the art. These families include amino acids with basic side chains (e.g., lysine, arginine, histidine), amino acids with acidic side chains (e.g., aspartic acid, glutamic acid), amino acids with uncharged polar side chains (e.g., glycine, asparagine, glutamine, serine, threonine, tyrosine, cysteine, tryptophan), amino acids with nonpolar side chains (e.g., alanine, valine, leucine, isoleucine, proline, phenylalanine, methionine), amino acids with β-branched side chains (e.g., threonine, valine, isoleucine), and amino acids with aromatic side chains (e.g., tyrosine, phenylalanine, tryptophan, histidine).

According to an embodiment of this application, the amino acid used for substitution or addition is selected from an amino acid X having a side chain containing -NH₂, -SH, -OH, or -COOH.

According to an embodiment of this application, the amino acid used for substitution or addition is selected from an amino acid X having a side chain containing -NH₂. Thus, additional modifiable sites are introduced into the original peptide, and the side-chain -NH₂ at these modification sites can bind to a modifying group, thereby effectively prolonging the in-vivo half-life of the peptide or the derivative or pharmaceutically acceptable salt thereof.

Herein, the term "modifying group" is to be understood broadly; it may be a chemical group or an amino acid fragment, and its specific type is not limited, all of which fall within the scope of protection of this application.

According to an embodiment of this application, the modifying group is linked to -NH₂ of the side chain of amino acid K in the peptide, or amino acid X after substitution or addition; the modifying group has at least one of the following structures:

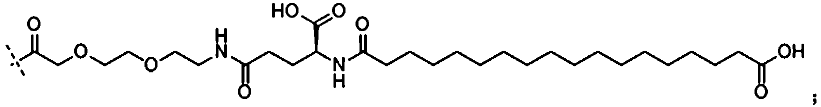

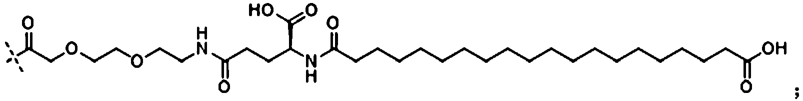

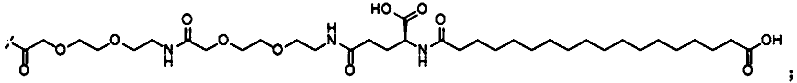

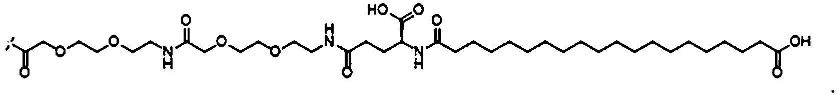

Herein, in the description of chemical groups, the symbol 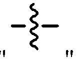 is used for indicating the position of group substitution. That is, the aforementioned chemical group is linked to -NH₂ of the amino acid through 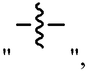 forming a -CO-NH- linkage.

According to an embodiment of this application, the amino acid X is selected from K, k, αMeK, HoK, Dap, Dab, Orn, or N-Me-K.

### Use

According to a second aspect of this application, this application provides use of the peptide or the derivative or pharmaceutically acceptable salt thereof according to the first aspect in preparation of a GLP-1R and GCGR dual-target agonist. Accordingly, the peptide or the derivative or pharmaceutically acceptable salt thereof is effective in activating GLP-1R and GCGR. Accordingly, the peptide or the derivative or pharmaceutically acceptable salt thereof can be prepared as a GLP-1R and GCGR dual-target agonist for treating or preventing a GLP-1R and/or GCGR-related disease, including, but not limited to, metabolic disorder-related diseases, such as dyslipidemia-related disease, for example, metabolic syndrome, especially obesity, diabetes, fatty liver disease, and non-alcoholic fatty liver disease.

### Fusion Protein, Reagent or Kit, and Pharmaceutical Composition

In a third aspect of this application, this application provides a fusion protein. According to an embodiment of this application, the fusion protein includes the peptide or the derivative or pharmaceutically acceptable salt thereof according to the first aspect. Accordingly, the peptide or the derivative or pharmaceutically acceptable salt thereof can be used for, through binding to GLP-1R and GCGR, effectively activating GLP-1R and GCGR. Accordingly, the fusion protein containing the peptide or the derivative or pharmaceutically acceptable salt thereof can be used for, through binding to GLP-1R and GCGR, detecting GLP-1R and/or GCGR or activating GLP-1R and GCGR, as well as treating or preventing GLP-1R and/or GCGR-related diseases, including, but not limited to, metabolic disorder-related diseases, such as dyslipidemia-related disease, for example, metabolic syndrome, especially obesity, diabetes, fatty liver disease, and non-alcoholic fatty liver disease.

In an exemplary embodiment of this application, the fusion protein further includes a functional fragment.

Herein, the term "functional fragment" refers to an amino acid fragment, which may be a functionally active fragment or a protein tag, and its specific type is not limited, all of which fall within the scope of protection of this application.

The aforementioned functionally active fragment may function in vivo in an animal or may function in vitro. Exemplarily, when the functionally active fragment functions in vivo in an animal, it may be used for preventing and/or treating a disease; when the functionally active fragment functions in vitro, it may be used for specifically binding a substance, for detecting the substance, or for in-vitro diagnosis of a disease.

The aforementioned protein tag refers to a short peptide expressed together with the protein of interest, which facilitates the expression, detection, tracing, or purification of the peptide of this application, and the like. Exemplarily, the protein tag includes at least one of His-tag, Flag-tag, GST-tag, MBP-tag, SUMO-tag, and C-Myc-tag.

In a fourth aspect of this application, this application provides a reagent or kit. According to an embodiment of this application, the reagent or kit includes the peptide or the derivative or pharmaceutically acceptable salt thereof according to the first aspect, or the fusion protein according to the third aspect. Accordingly, the peptide or the derivative or pharmaceutically acceptable salt thereof can be used for, through binding to GLP-1R and GCGR, effectively activating GLP-1R and GCGR. Accordingly, the reagent or kit containing the peptide or the derivative or pharmaceutically acceptable salt thereof can be used for, through binding to GLP-1R and GCGR, detecting GLP-1R and/or GCGR.

Herein, the kit or reagent need not have a physical box configuration; it only requires being relatively self-contained with suitable packaging or a container, such as a tube, a box, a bottle, or a card. Certain components are provided in separate containers, and, where permissible, some components may be combined in a single container.

According to an embodiment of this application, the kit includes a reagent suitable for performing the detection. In some implementations, the kit may contain instructions for performing the detection. In some implementations, the kit may contain a calibrator or control, such as standard or control sample. In some implementations, the kit further includes a container contained therein, such as a test tube, a microplate, or a test strip.

In a fifth aspect of this application, this application provides a pharmaceutical composition. According to an embodiment of this application, the pharmaceutical composition includes the peptide or the derivative or pharmaceutically acceptable salt thereof according to the first aspect, or the fusion protein according to the third aspect. Accordingly, the peptide or the derivative or pharmaceutically acceptable salt thereof can be used for, through binding to GLP-1R and GCGR, effectively activating GLP-1R and GCGR. Accordingly, the pharmaceutical composition including the peptide can be used for treating or preventing GLP-1R and/or GCGR-related diseases, including, but not limited to, metabolic disorder-related diseases, such as dyslipidemia-related disease, for example, metabolic syndrome, especially obesity, diabetes, fatty liver disease, and non-alcoholic fatty liver disease.

According to an embodiment of this application, the pharmaceutical composition may further include a pharmaceutically acceptable excipient.

In an exemplary embodiment of this application, the pharmaceutically acceptable excipient refers to a conventional pharmaceutical excipient in the pharmaceutical field, such as a diluent, a buffer, an osmotic pressure regulator, a pH adjuster, and the like.

In an exemplary embodiment of this application, a pharmaceutically acceptable carrier refers to a conventional pharmaceutical carrier in the pharmaceutical field, such as a protective agent, and the like.

In an exemplary embodiment of this application, a pharmaceutically acceptable vehicle refers to a conventional pharmaceutical vehicle in the pharmaceutical field, such as a solution (e.g., water) and liposome.

In an exemplary embodiment of this application, examples of suitable pharmaceutically acceptable carriers, excipients, and vehicles are well known in the art. A pharmaceutical composition including such carriers, excipients, and vehicles may be formulated by known conventional methods.

In some exemplary implementations of this application, the pharmaceutical composition may be an oral dosage form, such as a solid oral dosage form or a liquid oral dosage form, and the specific type is not limited, all of which fall within the scope of protection of this application.

In some exemplary implementations of this application, the pharmaceutical composition in this application may further contain other active ingredients for treatment.

The pharmaceutical composition in this application may be administered by various routes, such as enterally, orally (e.g., as a liquid solution), or via injection (e.g., intravenous, subcutaneous, intramuscular, intraperitoneal, intradermal). Preferably, the pharmaceutical composition in this application is in the form of a lyophilized formulation or an aqueous solution. The clinical dosing regimen will be determined by an attending physician and clinical factors. As is well known in the medical field, the dose for any individual patient depends on many factors, including the patient's physique, body surface area, age, the medicament to be administered, gender, time and route of administration, general health, and other medicaments administered concurrently. The pharmaceutical composition in this application may be administered locally or systemically. Preferably, it may be administered intravenously or subcutaneously.

### Use

In a sixth aspect of this application, this application provides use of the peptide or the derivative or pharmaceutically acceptable salt thereof according to the first aspect, the fusion protein according to the third aspect, or the pharmaceutical composition according to the fifth aspect in preparation of a medicament for treating or preventing a GLP-1R and/or GCGR-related disease, such as metabolic disorder-related disease.

This application provides use of the peptide or the derivative or pharmaceutically acceptable salt thereof according to the first aspect, the fusion protein according to the third aspect, or the pharmaceutical composition according to the fifth aspect in treatment or prevention of a GLP-1R and/or GCGR-related disease.

This application provides use of the peptide or the derivative or pharmaceutically acceptable salt thereof according to the first aspect, the fusion protein according to the third aspect, or the pharmaceutical composition according to the fifth aspect in treatment or prevention of a GLP-1R and/or GCGR-related disease.

According to an embodiment of this application, the above uses may further include at least one of the following technical features:
According to an embodiment of this application, the GLP-1R and/or GCGR-related disease includes a metabolic disorder-related disease.

According to an embodiment of this application, the metabolic disorder-related disease includes a dyslipidemia-related disease.

According to an embodiment of this application, the dyslipidemia-related disease includes a metabolic syndrome.

According to an embodiment of this application, the metabolic disorder-related disease includes at least one of obesity, diabetes, fatty liver disease, and non-alcoholic fatty liver disease.

### Methods

In a seventh aspect of this application, this application provides a method for detecting GLP-1R and/or GCGR. According to an embodiment of this application, the method includes: contacting a sample to be detected with the peptide or the derivative or pharmaceutically acceptable salt thereof according to the first aspect, the fusion protein according to the third aspect, or the reagent or kit according to the fourth aspect; and determining whether the sample to be detected contains GLP-1R and/or GCGR based on a signal generated from a contact product. Accordingly, the peptide or the derivative or pharmaceutically acceptable salt thereof can be used for, through binding to GLP-1R and GCGR, effectively activating GLP-1R and GCGR. Accordingly, the method can be used for, through binding to GLP-1R and GCGR, detecting GLP-1R and/or GCGR.

According to a specific embodiment of this application, the signal includes a fluorescence signal. According to a specific embodiment of this application, the method further includes determining a content value of GLP-1R and/or GCGR in the sample to be detected based on the signal generated from the contact product.

In an eighth aspect of this application, this application provides a method for treating or preventing a metabolic disorder-related disease. According to an embodiment of this application, the method includes: administering to a subject a pharmaceutically acceptable dose of the peptide or the derivative or pharmaceutically acceptable salt thereof according to the first aspect, the fusion protein according to the third aspect, or the pharmaceutical composition according to the fifth aspect.

In an exemplary embodiment of this application, the pharmaceutically acceptable dose may be selected from an effective dose (also referred to as an effective amount).

The effective amount of the peptide or the derivative or pharmaceutically acceptable salt thereof in this application may vary depending on the mode of administration, the severity of the disease to be treated, and other factors. The selection of a preferred effective amount may be determined by those of ordinary skill in the art based on various factors (e.g., through clinical trials). Such factors include, but are not limited to, pharmacokinetic parameters of the active ingredient such as bioavailability, metabolism, half-life, and the like; the severity of the disease to be treated in the patient, the patient's body weight, the patient's immune status, the route of administration, and the like. For example, depending on the urgency of the treatment condition, several divided doses may be administered daily, or the dose may be proportionally reduced.

The peptide or the derivative or pharmaceutically acceptable salt thereof in this application, or the pharmaceutical composition thereof can be incorporated into suitable medicaments. These medicaments may be prepared in various forms, such as liquid, semisolid, and solid dosage forms, including but not limited to solid dosage forms, semisolid dosage forms, liquid dosage forms, and gaseous dosage forms, and the like. Various routes of administration of the peptide or the derivative or pharmaceutically acceptable salt thereof in this application, or the pharmaceutical composition or medicament thereof are contemplated, including peritoneal, intravenous, intramuscular, subcutaneous, dermal, oral, topical, nasal, pulmonary, rectal, and topical application, but this application is not limited to these exemplified routes of administration.

According to an embodiment of this application, the metabolic disorder-related disease includes a dyslipidemia-related disease.

According to an embodiment of this application, the dyslipidemia-related disease includes a metabolic syndrome.

According to an embodiment of this application, the metabolic disorder-related disease includes at least one of obesity, diabetes, fatty liver disease, and non-alcoholic fatty liver disease.

The embodiments of this application will be explained below with reference to the embodiments. A person skilled in the art will understand that the following embodiments are provided only to describe this application and are not to be construed as limiting the scope of this application. Where specific techniques or conditions are not indicated in the embodiments, they are carried out according to the techniques or conditions described in the literature in the field or according to the product instructions. Reagents or instruments for which the manufacturer is not specified are conventional products available through commercial purchase.

### Embodiment 1:

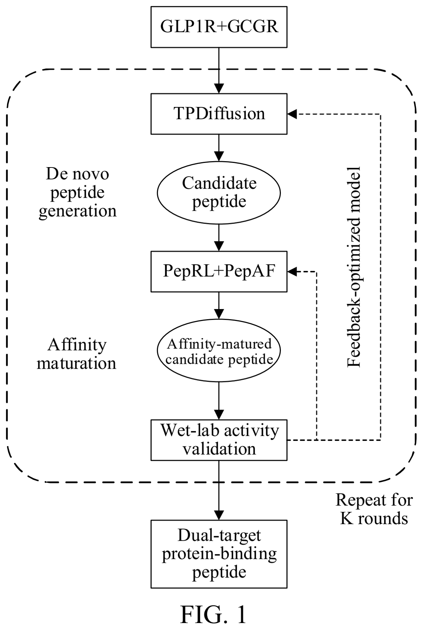

A schematic diagram of a peptide design and screening process of this application is shown in FIG. 1, which outlines a series of operations from the generation of GLP-1R and GCGR dual-target peptide sequences to their screening and optimization. The overall method was composed of multiple iterative evolution cycles, with each iterative evolution cycle yielding a designed peptide exhibiting enhanced biological activity. Each iterative evolution included three parts: AI-based de novo peptide generation and optimization, wet-lab experiments to determine peptide bio-activity, and feedback to reinforce the model's perception of bio-activity.

First, a novel deep-learning method named TPDiffusion enabled the generation of peptide sequences capable of binding to a target protein based on its amino acid sequence. This method, which translated the target protein sequence into a peptide sequence, might be viewed as a process of generating specific answers to given questions. By training the conditional diffusion model TPDiffusion to learn the relational rules between target proteins and peptide sequences, it achieved the generation of specific peptides (i.e., GLP-1R and GCGR dual-target peptides) for given target proteins (i.e., GLP-1R and GCGR). The training of the peptide sequence generation model TPDiffusion primarily involved a forward diffusion process and a reverse diffusion process. The forward diffusion process included the following operations:
1. Encode amino acid sequences: To model the joint feature space of protein-peptide, the target protein sequence and the peptide sequence were concatenated into a single entity. An embedding transformation function was introduced to map each discrete amino acid token or character into a continuous vector encoding space.
2. Gradually add noise to the peptide sequence: Gaussian noise was progressively added to the peptide portion of the vector encoding based on a Markov chain until the peptide sequence was completely corrupted.

After completion of the forward diffusion process, a set of clean target protein sequences and noisy peptide sequences was obtained. Subsequently, the peptide sequence was reconstructed through a reverse diffusion process, which mainly included the following operations:
1. Gradually denoise the peptide sequence: A denoising network was constructed to estimate the noise distribution added during the forward diffusion process, and the peptide portion was progressively denoised until the peptide sequence was fully recovered.
2. Calculate a loss function: The model outputted a predicted probability distribution of the peptide sequence, and a mean squared error loss function was used for computing the difference between the model's predictions and the true results, followed by backpropagation to update the model parameters.

The denoising network in TPDiffusion employed the BERT model (Devlin et al., 2018). The architecture of BERT was mainly composed of multiple layers of Transformer encoders (Vaswani et al., 2017). Each Transformer block included two parts: a self-attention mechanism and a feed-forward neural network. Through the self-attention mechanism, TPDiffusion incorporated target sequence information during the reverse-diffusion-based recovery of the peptide sequence, thereby implicitly modeling the relational features between the target protein and the peptide sequence and achieving a mapping from the target protein to the peptide sequence. In the generation process, given any target protein sequence, the model first randomly sampled from Gaussian noise and then performed the reverse diffusion process. Guided by the target protein sequence, the noise was progressively eliminated over fixed time steps, ultimately generating a binding peptide sequence specific to the given target. Using the trained TPDiffusion model, with the sequences of both GLP-1R and GCGR as input, a batch of candidate peptide sequences potentially exhibiting high affinity for both GLP-1R and GCGR targets was generated.

Second, affinity maturation was performed on these high-affinity candidate peptide sequences. This method was an innovative deep reinforcement learning scheme specifically designed for optimizing peptide sequences. It guided the evolution of candidate peptide sequences by simulating an environment and defining actions. The deep reinforcement learning framework might incorporate some reward models as prior knowledge to guide peptide evolution. The reward model might be an affinity prediction model, a solubility prediction model, a toxicity prediction model, or the like.

The reward model was an innovative technical solution termed PepAF, which effectively predicted the binding affinity between a target protein and a peptide by comprehensively utilizing structural information, flexibility characteristics, and advanced pre-training strategies. PepAF first learned from two pre-training tasks: 1) estimating the binding free energy of protein (GLP-1R and GCGR)-peptide complexes; and 2) predicting the affinity of protein (GLP-1R and GCGR)-peptide complexes. The first task enabled the model to learn the complex interactions between the protein (GLP-1R and GCGR) and the peptide, as well as their structural and physicochemical properties, thereby providing a foundation for understanding the binding patterns and key features of protein (GLP-1R and GCGR)-peptide interactions. The second task enabled the model to capture a wide range of molecular interactions at the atomic scale. PepAF further improved prediction accuracy by modeling the structure of the target protein and the flexibility of the peptide. The core technology PepAF from this patent was employed as the reward model in the deep reinforcement learning method to guide mutations in candidate peptide sequences.

After obtaining affinity-matured peptides, their bio-activity was evaluated, and the results were fed back to the model to proceed with the next round of peptide generation and optimization. After one iteration, a peptide for effectively activating both target proteins, GLP-1R and GCGR, was designed. Its sequence is set forth in SEQ ID NO: 1. The amino acid sequence of SEQ ID NO: 1 is as follows:

| Peptide name | Amino acid sequence | SEQ ID NO: |
|---|---|---|
| GA-Dual.R5.S3 | HSEGTFTSDYSKYLEEEAAALFIAWLLAGG | 1 |

### Embodiment 2: Evaluation of Activating Ability of Candidate Peptide on both GLP-1R and GCGR Target Proteins

1. This embodiment is designed to evaluate the cAMP-activating ability of the candidate peptide (GA-Dual.R5.S3) obtained in Embodiment 1 on overexpressed huGLP-1R-FL-CRE-HEK293-A5 cells. By quantitatively measuring the level of cAMP generation, this experiment can precisely evaluate the activating effect of the candidate sample on the GLP-1R receptor, providing important bio-activity data for medicament development in metabolic diseases such as diabetes. The experimental process was as follows:
   Peptide synthesis and purification: According to the designed specific amino acid sequences, the candidate peptides were synthesized using solid-phase peptide synthesis (Fmoc-SPPS) technology. The purification process employed high-performance liquid chromatography (HPLC) to ensure that the synthesized peptides were of high purity with no significant impurities. Further, the exact molecular weight of the peptides was verified by mass spectrometry (MS).

Peptide dissolution conditions: The purified candidate peptides were each dissolved in a DMSO (dimethyl sulfoxide) solvent to prepare 1 mg/mL stock solutions for subsequent experiments.

Cell starvation treatment: huGLP-1R-FL-CRE-HEK293-A5 cell lines were constructed. Highly viable huGLP-1R-FL-CRE-HEK293-A5 were selected and subjected to starvation in a DMEM medium containing 0% fetal bovine serum (FBS) to standardize experimental conditions and eliminate interference from other serum factors.

Cell digestion, washing, and plating: Adherent cells were gently digested using Accutase enzyme and washed with PBS buffer to remove the residual medium and enzyme. The cell suspension was precisely plated into an HTRF 96-well low-volume assay plate to ensure consistent cell numbers per well, thereby providing a uniform starting condition for the subsequent cAMP activity assay.

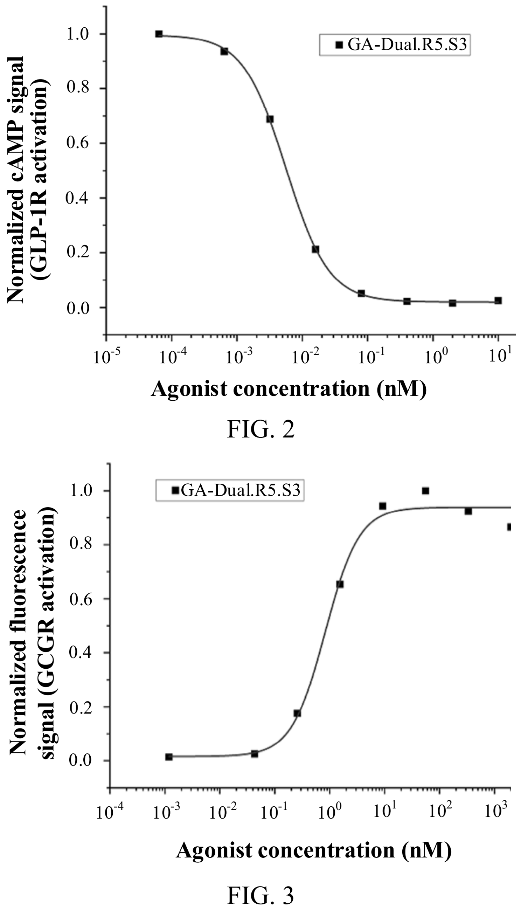

cAMP activity assay: After plating, a precisely diluted candidate peptide (specific concentrations are indicated on the x-axis of FIG. 2) was added to each well, followed by incubation at 37°C for 20 minutes to mimic the in-vivo environment and activate the GLP-1R and GCGR receptors. cAMP d2 reagent and an anti-cAMP Eu Cryptate antibody (PerkinElmer, Cat. No. 62AM4PEB) working solution were added to each well, followed by incubation at room temperature for 1 hour. This operation allowed cAMP to bind to the fluorescently labeled antibody, forming an energy-transfer complex.

Signal detection: Using TR-FRET technology, an excitation wavelength of 340 nm was set, and emission signals were detected at 665 nm and 620 nm, respectively. This technique offered high sensitivity and strong anti-interference capability. The FRET signal value was calculated as the ratio of the light intensity at 665 nm to that at 620 nm, reflecting changes in cAMP concentration. The change in the ratio was inversely proportional to the cAMP concentration.

The results indicated that the synthesized candidate peptides all exhibited good cAMP-activating activity, demonstrating that the candidate peptides possessed high GLP-1R activating bio-activity. The detection results are shown in FIG. 2, and the EC₅₀ values of some candidate peptides are listed in Table 1.

**Table 1**

| Peptide name | Amino acid sequence | EC₅₀ (pM) |
|---|---|---|
| GA-Dual.R5.S3 | HSEGTFTSDYSKYLEEEAAALFIAWLLAGG | 5.6 |

2. This embodiment was designed to evaluate the activating ability of the candidate peptide (GA-Dual.R5.S3) obtained in Embodiment 1 on the GCGR target. The experimental process was as follows:
First, the candidate peptide was serially diluted using a complete DMEM medium to prepare a series of concentration gradients. Then, 50 µL of each dilution was added to the culture plate. Human GCGR-CRE-luci-HEK293-A5 cells were added at a density of 1×10⁵ cells per well in 50 µL volume to the culture plate and mixed with the diluted agonist. The culture plate was placed in a 37°C incubator for 6 hours to ensure adequate cellular uptake of the agonist and activation of GCGR. Then, 50 µL of Bright-Lite reagent (Vazyme, Cat. No. DD1204-04-AA) was added to each well, and the luciferase signal was read using a luciferase detector to evaluate GCGR activation.

The results show that the synthesized candidate peptide exhibits good luciferase-activating activity, indicating that the candidate peptide possesses high GCGR-activating bio-activity. The detection results are shown in FIG. 3, and the EC₅₀ values of some candidate peptides are listed in Table 2.

**Table 2**

| Peptide name | Amino acid sequence | EC₅₀ (nM) |
|---|---|---|
| GA-Dual.R5.S3 | HSEGTFTSDYSKYLEEEAAALFIAWLLAGG | 0.83 |

### Embodiment 3:

This embodiment was designed to evaluate the therapeutic effects of the candidate peptide (GA-Dual.R5.S3) from Embodiment 1 in vitro on obesity, diabetes, dyslipidemia-related diseases, fatty liver disease, metabolic syndrome, and non-alcoholic fatty liver disease, respectively. The specific operations were as follows:
1) For obesity: 3T3-L1 preadipocytes were used. The cells were first induced to differentiate into adipocytes using a high-glucose DMEM medium (containing 4.5 g/L glucose, supplemented with 10% fetal bovine serum, 100 U/mL penicillin, and 100 µg/mL streptomycin; Thermo Fisher Scientific, Cat. No. 11995065) to establish an obesity model. When the confluency of 3T3-L1 preadipocytes reached 80% to 90%, the cells were digested with 0.25% trypsin and seeded at a density of 2×10⁴ cells/cm² to 3×10⁴ cells/cm². When the cell confluency reached 80% to 95%, an adipogenic induction differentiation medium was added to initiate differentiation. After 2 days to 3 days of induction, the medium was replaced with an adipogenic differentiation maintenance medium for 1 day. This induction-maintenance cycle was repeated 3 times to 5 times until prominent lipid droplets were observed inside the cells, indicating successful establishment of the obesity model.
   The candidate peptide was then added to the adipocyte culture medium at different concentrations (10 nM, 100 nM, 1 µM, and 10 µM). The cell density was maintained at 2×10⁴ cells/cm² to 3×10⁴ cells/cm² to ensure normal cell growth and differentiation, followed by culture for 24 hours to 48 hours. Intracellular lipid droplet content was detected by Oil Red O staining: The medium was removed, and the cells were washed once with 1× PBS. Then, 4% neutral formaldehyde solution was added to fix the cells for 30 min. An Oil Red O working solution was prepared by mixing saturated Oil Red O stock solution with distilled water at a ratio of 3:2, filtered, and used for staining. 1 mL of the Oil Red O working solution was added to each well, and staining was performed at room temperature for 30 min. The staining solution was then aspirated, and the cells were washed with 1× PBS. The results were observed and photographed under a microscope. Concurrently, a qPCR kit (Takara RR420A) was used to detect the expression levels of adipogenesis-related genes (PPARγ, C/EBPα). If the candidate peptide can significantly reduce intracellular lipid droplet content and down-regulate the expression of adipogenesis-related genes, it indicates potential therapeutic efficacy against obesity, possibly by inhibiting adipocyte differentiation and lipid accumulation.
2) For diabetes: A pancreatic β-cell line (MIN6 cells) was employed. The cells were cultured in high-glucose DMEM medium (containing 4.5 g/L glucose, supplemented with 10% fetal bovine serum, 100 U/mL penicillin, and 100 µg/mL streptomycin; Thermo Fisher Scientific, Cat. No. 11995065) to simulate the hyperglycemic environment of diabetes. The candidate peptide was added to the cell culture medium at different concentrations (10 nM, 100 nM, 1 µM, and 10 µM). The cell density was maintained at 5×10³ cells/cm², followed by culture for 24 hours to 48 hours. An insulin ELISA kit was used for measuring insulin secretion in the cell culture supernatant. Meanwhile, a GLUT2 kit (YUANJU BIO, Cat. No. YJ24519) and an IRS1 kit (Thermo Fisher Scientific, Cat. No. KHO0521) were used for detecting the expression levels of proteins related to the insulin signaling pathway, such as the glucose transporter (GLUT2) and insulin receptor substrate (IRS). If the candidate peptide can increase insulin secretion and up-regulate the expression levels of GLUT2 and IRS, it suggests that the candidate peptide may improve insulin resistance and promote insulin secretion, indicating potential value for the treatment of diabetes.
3) For dyslipidemia-related diseases: A hepatocyte line (HepG2 cells) was selected. The cells were treated with a medium containing high concentrations of fatty acids (HepG2 cell-specific medium from Wuhan Sunncell Biotechnology Co., Ltd., including EMEM (MEM + NEAA) for cell treatment, supplemented with 10% fetal bovine serum (FBS) and 1% penicillin/streptomycin (P/S)) to establish a dyslipidemia model. The candidate peptide was added to the cell culture medium at different concentrations (10 nM, 100 nM, 1 µM, and 10 µM). The cell density was maintained at 5×10³ cells/cm², followed by culture for 24 hours to 48 hours. An enzymatic assay kit (Sigma-Aldrich Total Cholesterol Assay Kit, Cat. No. MAK043) was used for measuring intracellular total cholesterol content, and a triglyceride assay kit (Sigma-Aldrich Triglyceride Assay Kit, Cat. No. MAK041) was used for measuring triglyceride content. Meanwhile, an LDLR kit (Thermo Fisher Scientific, Cat. No. EHLDLR) and a CYP7A1 kit (proteintech, Cat. No. 18054-1-AP) were used for detecting the expression levels of genes related to cholesterol metabolism, such as the low-density lipoprotein receptor (LDLR) and cholesterol 7α-hydroxylase (CYP7A1). If the candidate peptide can reduce intracellular total cholesterol and triglyceride levels while up-regulating the expression of LDLR and CYP7A1, it indicates that the candidate peptide may exert a therapeutic effect on dyslipidemia-related diseases by modulating cholesterol synthesis and metabolism.
4) For fatty liver disease: Similarly, HepG2 cells were used. The cells were induced to develop steatosis using a high-fat medium (HepG2 cell-specific medium from Wuhan Sunncell Biotechnology Co., Ltd., including EMEM (MEM + NEAA) supplemented with 10% fetal bovine serum (FBS) and 1% penicillin/streptomycin (P/S)) to establish a fatty liver disease model. The candidate peptide was added to the cell culture medium at different concentrations (10 nM, 100 nM, 1 µM, and 10 µM). The cell density was maintained at 5×10³ cells/cm², followed by culture for 24 hours to 48 hours. Intracellular lipid droplet content was detected by using Oil Red O staining: The medium was removed, and the cells were washed once with 1× PBS. Then, 4% neutral formaldehyde solution was added to fix the cells for 30 min. An Oil Red O working solution was prepared by mixing saturated Oil Red O stock solution with distilled water at a ratio of 3:2, filtered, and used for staining. 1 mL of the Oil Red O working solution was added to each well, and staining was performed at room temperature for 30 min. The staining solution was then aspirated, and the cells were washed with 1× PBS. The results were observed and photographed under a microscope. Meanwhile, ELISA kits (TNF-α and IL-6 ELISA kits from Thermo Fisher Scientific, Cat. Nos. K1480 and K1501, respectively) were used for measuring the secretion levels of inflammatory factors. Colorimetric assay kits (MDA Assay Kit, Cat. No. 700455, and SOD Assay Kit, Cat. No. 705125, from Cayman Chemical) were employed to detect oxidative stress markers. If the candidate peptide can reduce intracellular lipid droplet accumulation, decrease the secretion of inflammatory factors, and ameliorate oxidative stress, it suggests that the candidate peptide may have a therapeutic effect on fatty liver disease, potentially alleviating hepatocellular steatosis and inflammatory injury.
5) For metabolic syndrome: A cellular model of metabolic syndrome was constructed by combining multiple cell types (e.g., adipocytes, hepatocytes, myocytes) to simulate the complex pathological environment of metabolic syndrome.

Adipocytes: 3T3-L1 preadipocytes were induced to differentiate into adipocytes using a high-glucose DMEM medium (containing 4.5 g/L glucose, supplemented with 10% fetal bovine serum, 100 U/mL penicillin, and 100 µg/mL streptomycin) to establish an obesity model.

Hepatocytes: HepG2 cells were treated with a high-fat medium (EMEM supplemented with 10% fetal bovine serum, 1% penicillin/streptomycin, and added high-concentration fatty acids) to simulate a fatty liver disease environment.

Myocytes: C2C12 myocytes were cultured in a high-glucose DMEM medium to simulate an insulin-resistant environment.

The candidate peptide was added to the cell culture medium at different concentrations (10 nM, 100 nM, 1 µM, and 10 µM). The cell density was maintained at 5×10³ cells/cm², followed by culture for 24 hours to 48 hours. After culturing, relevant indicators associated with metabolic syndrome were detected in each cell type: the insulin resistance index (HOMA-IR, calculated based on detected insulin and glucose levels); blood lipid levels (triglyceride and total cholesterol levels were measured using the Triglyceride Assay Kit (Sigma-Aldrich, Cat. No. MAK041) and the Total Cholesterol Assay Kit (Sigma-Aldrich, Cat. No. MAK043), respectively); and inflammatory factors (secretion levels of TNF-α and IL-6 were measured using ELISA kits (TNF-α and IL-6 ELISA kits from Thermo Fisher Scientific, Cat. Nos. K1480 and K1501, respectively)), and the like. If the candidate peptide can improve insulin resistance, reduce blood lipid levels, and decrease the secretion of inflammatory factors, it indicates potential therapeutic efficacy against metabolic syndrome, likely through a multi-target mechanism to comprehensively regulate metabolic disorders.

6) For non-alcoholic fatty liver disease (NAFLD): HepG2 cells were used. The cells were induced to develop NAFLD-like alterations using a high-fat/high-glucose medium (HepG2 cell-specific medium from Wuhan Sunncell Biotechnology Co., Ltd., including EMEM (MEM + NEAA) for cell treatment, supplemented with 10% fetal bovine serum (FBS) and 1% penicillin/streptomycin (P/S)). The candidate peptide was added to the cell culture medium at different concentrations (10 nM, 100 nM, 1 µM, and 10 µM). The cell density was maintained at 5×10³ cells/cm², followed by culture for 24 hours to 48 hours. Intracellular lipid droplet content was measured by using Oil Red O staining. Meanwhile, ELISA kits (IL-1β and IL-8 ELISA kits from Thermo Fisher Scientific, Cat. Nos. K1480 and K1501, respectively) were used for measuring the secretion levels of inflammatory factors. Colorimetric assay kits (ALT Assay Kit, Cat. No. 700455, and AST Assay Kit, Cat. No. 705125, from Cayman Chemical) were used for detecting the levels of hepatocellular injury markers. Further, Western blot and qPCR were used for detecting the expression levels of markers (collagen I and α-smooth muscle actin) related to liver fibrosis. If the candidate peptide can reduce intracellular fat content, decrease the secretion of inflammatory factors, lower the levels of hepatocellular injury markers, and suppress the expression of markers related to liver fibrosis, it indicates that the candidate peptide possesses a certain therapeutic effect against non-alcoholic fatty liver disease, likely by mitigating fat accumulation and inhibiting inflammatory and fibrotic processes.

The results show that the candidate peptides in Embodiment 1 exhibit the following effects:
1) They all can significantly reduce lipid droplet content in 3T3-L1 adipocytes and down-regulate the expression of adipogenesis-related genes.
2) They all can increase insulin secretion in pancreatic β-cell lines (e.g., MIN6 cells) and up-regulate the expression of proteins related to the insulin signaling pathway, such as the glucose transporter (GLUT2) and insulin receptor substrate (IRS).
3) They can lower the total cholesterol and triglyceride levels in hepatocyte lines while up-regulating the expression of genes related to cholesterol metabolism, such as the low-density lipoprotein receptor (LDLR) and cholesterol 7α-hydroxylase (CYP7A1).
4) They can reduce lipid droplet accumulation in HepG2 cells, decrease the secretion of inflammatory factors, and improve the oxidative stress status.
5) They can improve indicators related to metabolic syndrome in multiple cell types (e.g., adipocytes, hepatocytes, myocytes).
6) They can decrease fat content in HepG2 cells, reduce the secretion of inflammatory factors, lower the levels of hepatocellular injury markers, and suppress the expression of markers related to liver fibrosis.

In summary, the above results demonstrate that the candidate peptide GA-Dual.R5.S3 has therapeutic effects against obesity, diabetes, dyslipidemia-related diseases, fatty liver disease, metabolic syndrome, and non-alcoholic fatty liver disease.

In the descriptions of this specification, a description of a reference term such as "an embodiment", "some embodiments", "an example", "a specific example", or "some examples" means that a specific feature, structure, material, or characteristic that is described with reference to the embodiment or the example is included in at least one embodiment or example of this application. In this specification, the schematic descriptions of the foregoing terms are not necessarily directed at the same embodiment or example. Besides, the specific feature, structure, material, or characteristic that is described may be combined in proper manners in any one or more embodiments or examples. In addition, a person skilled in the art may integrate or combine different embodiments or examples described in the specification and features of the different embodiments or examples as long as they are not contradictory to each other.

Although the embodiments of this application have been shown and described above, the foregoing embodiments are exemplary and are not to be construed as limiting this application. Those of ordinary skill in the art can make variations, modifications, substitutions, and adaptations to the above embodiments within the scope of this application.

## Claims

1. A peptide or a derivative or pharmaceutically acceptable salt thereof, an amino acid sequence of the peptide being set forth in SEQ ID NO: 1.

2. The peptide or the derivative or pharmaceutically acceptable salt thereof according to claim 1, wherein the derivative of the peptide comprises a modifying group, and the peptide is linked to the modifying group.

3. The peptide or the derivative or pharmaceutically acceptable salt thereof according to claim 2, wherein the modifying group is linked to -NH₂ of an amino acid side chain in the peptide; and/or the modifying group has at least one of the following structures:

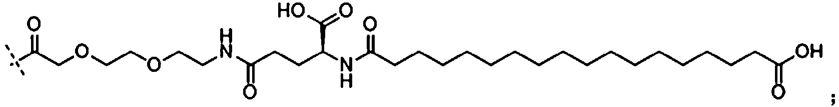

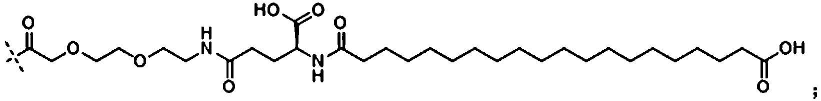

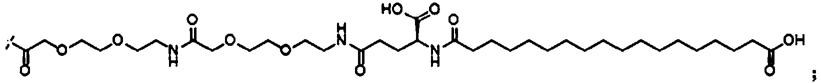

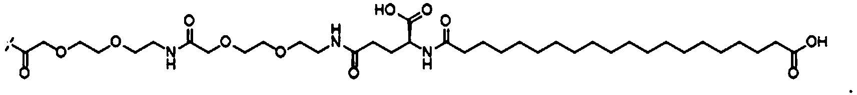

4. The peptide or the derivative or pharmaceutically acceptable salt thereof according to any one of claims 1 to 3 for use in preparation of a GLP-1R and GCGR dual-target agonist.

5. The peptide or the derivative or pharmaceutically acceptable salt thereof according to any one of claims 1 to 3 for use in preparation of a medicament for treating or preventing a GLP-1R and/or GCGR-related disease.

6. A fusion protein, comprising the peptide or the derivative or pharmaceutically acceptable salt thereof according to any one of claims 1 to 3.

7. A reagent or kit, comprising the peptide or the derivative or pharmaceutically acceptable salt thereof according to any one of claims 1 to 3.

8. A pharmaceutical composition, comprising the peptide or the derivative or pharmaceutically acceptable salt thereof according to any one of claims 1 to 3; and optionally a pharmaceutically acceptable excipient.

9. The pharmaceutical composition according to claim 8 for use in the treating or preventing a GLP-1R and/or GCGR-related disease.

10. The pharmaceutical composition for use according to claim 9, wherein the GLP-1R and/or GCGR-related disease comprises a metabolic disorder-related disease.

11. The pharmaceutical composition for use according to claim 10, wherein the metabolic disorder-related disease comprises a dyslipidemia-related disease.

12. The pharmaceutical composition for use according to claim 11, wherein the dyslipidemia-related disease comprises a metabolic syndrome.

13. The pharmaceutical composition for use according to claim 12, wherein the metabolic syndrome comprises at least one of obesity, diabetes, fatty liver disease, and non-alcoholic fatty liver disease.

14. A method for detecting GLP-1R and/or GCGR, comprising:
contacting a sample to be detected with the peptide or the derivative or pharmaceutically acceptable salt thereof according to any one of claims 1 to 3, the fusion protein according to claim 5, or the reagent or kit according to claim 6; and
determining whether the sample to be detected contains GLP-1R and/or GCGR based on a signal generated from a contact product.
